# EUROPEAN PATENT APPLICATION

(11) **EP 1 203 771 A1**
(43) Date of publication of application: **08.05.2002**
(21) Application number: 00946400.9
(22) Date of filing: 19.07.2000
(51) Int. Cl.: C07D 489/09, A61K 31/485, A61P 13/02

(54) **REMEDIES OR PREVENTIVES FOR FREQUENT URINATION OR URINARY INCONTINENCE**

(30) Priority: 19.07.1999 JP 20480999
(71) Applicant: TORAY INDUSTRIES, INC., Tokyo 103-8666 (JP)
(72) Inventor: TANAKA, Toshiaki, Zushi-shi, Kanagawa 249-0004 (JP); NAGASE, Hiroshi, Kamakura-shi, Kanagawa 247-0063 (JP); ENDOH, Takashi, Chigasaki-shi, Kanagawa 253-0071 (JP); KAWAMURA, Kuniaki, Kamakura-shi, Kanagawa 248-0034 (JP); FUJIMURA, Morihiro, Aoba-ku, Yokohama-shi, Kanagawa 227-0048 (JP); KOMAGATA, Toshikazu, Kamakura-shi, Kanagawa 248-0031 (JP)
(74) Representative: Kador & Partner
(86) International application number: JP0004839
(87) International publication number: WO0105795

(57) **Abstract**

A therapeutic or prophylatic agent for frequent urination or urinary incontinence comprising as an effective ingredient a quinolinomorphinane derivative, for example, of the formula: 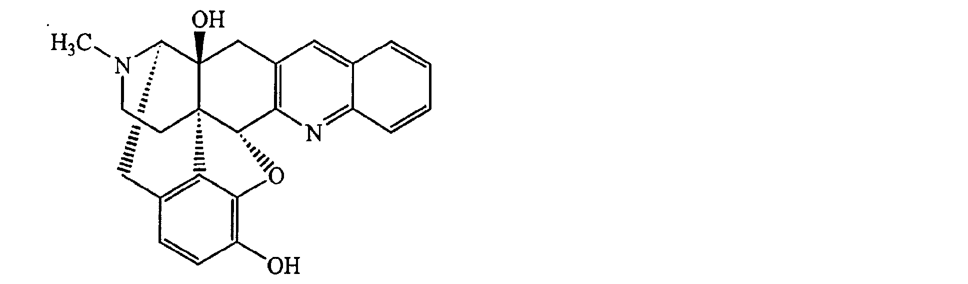 or a pharmaceutically acceptable acid addition salt thereof.

## Description

### Technical Field

The present invention relates to a therapeutic or prophylatic agent for frequent urination or urinary incontinence.

### Background Art

Recently, with coming of an aging society, the number of patients suffering from frequent urination or urinary incontinence is increasing year by year. At present, as therapeutic drugs for frequent urination or urinary incontinence, propiverine hydrochloride, oxybutynin hydrochloride and flavoxate hydrochloride, which have anticholinergic activities and activities to directly relaxing smooth muscle, are used.

However, these existing drugs often cause dry mouth, digestive system symptoms such as constipation, cardiovascular symptoms such as orthostatic hypotension, and dysuria such as ischuria and residual urine, as side effects.

Cerebrovascular diseases and dementia are thought to be the largest cause of dysuria in old people. Recently, it is concerned that by administering the existing drugs having anticholinergic activities for the therapy of frequent urination or urinary incontinence accompanied by encephalopathies, acetylcholine in the brain is inhibited, so that the encephalopathies *per se* progress.

The side effects of these existing drugs are not satisfactory in view of quality of life (QOL) of the patients, and so development of a therapeutic or prophylatic agent for frequent urination or urinary incontinence is strongly demanded.

On the other hands, prior art of quinolinomorphinane derivatives include U.S. Patent 4,816,586, International Publications WO94/14445 and WO98/43977. These patent references disclose analgesics, antitussives and therapeutic agents for encephalopathies, but do not disclose a therapeutic or prophylatic agent for frequent urination or urinary incontinence at all.

### Disclosure of the Invention

An object of the present invention is to provide a novel therapeutic or prophylatic agent for frequent urination or urinary incontinence, of which side effects are reduced.

The above-mentioned object is attained by the present invention, which follows:

That is, the present invention provides a therapeutic or prophylatic agent for frequent urination or urinary incontinence, comprising as an effective ingredient a quinolinomorphinane derivative of the Formula (I): 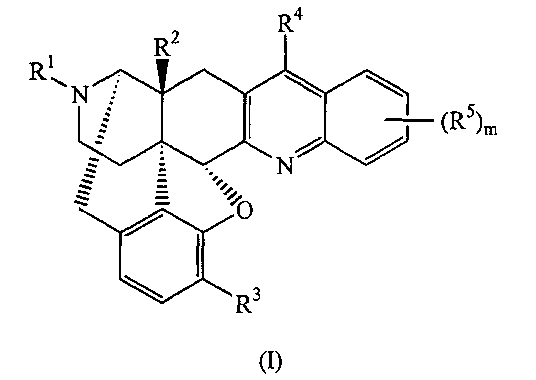 (wherein R¹ is hydrogen, C₁-C₅ alkyl, C₄-C₇ cycloalkylalkyl, C₅-C₇ cycloalkenylalkyl, C₆-C₁₂ aryl, C₇-C₁₃ aralkyl, C₂-C₇ alkenyl, C₁-C₅ alkanoyl, furan-2-ylalkyl (wherein the number of carbon atoms in the alkyl moiety is 1 to 5), or thiophene-2-ylalkyl (wherein the number of carbon atoms in the alkyl moiety is 1 to 5);
R² and R³ independently are hydrogen, hydroxy, C₁-C₅ alkoxy, C₁-C₅ alkanoyloxy, C₇-C₁₃ aralkyloxy or C₇-C₁₃ arylcarbonyloxy;
m is an integer of 0 to 4;
R⁵(s) is(are) substituent(s) on the benzene ring and independently represent(s) R¹⁸, or two R⁵s on adjacent carbon atoms cooperatively represent a fused ring structure A (with the proviso that the remaining 0 to 2 R⁵s independently represent R¹⁸ or another pair of R⁵s represent another fused ring structure A),
   wherein said ring structure A is benzo, indeno, naphtho or pyrido, or C₅-C₇ cycloalkeno, each of which is substituted with 0 to 4 R⁹s, or non-substituted dioxoleno;
R⁹ and R¹⁸ represent (1) independently fluoro, chloro, bromo, iodo, nitro, hydroxy, C₁-C₅ alkyl, C₁-C₅ alkoxy, isothiocyanato, trifluoromethyl, trifluoromethoxy, cyano, phenyl, C₁-C₃ hydroxyalkyl, SR⁶, SOR⁶, SO₂R⁶, (CH₂)ₖCO₂R⁷, SO₂NR⁷R⁸, CONR⁷R⁸, (CH₂)ₖNR⁷R⁸, or (CH₂)ₖN(R⁷)COR⁸ (wherein k is an integer of 0 to 5, R⁶ is C₁-C₅ alkyl, R⁷ and R⁸ independently are hydrogen, C₁-C₅ alkyl or C₄-C₆ cycloalkylalkyl), and/or (2) R⁹ and R¹⁸ on adjacent carbon atoms via the fused ring moiety cooperatively form R⁹-R¹⁸ which represents a bridging structure selected from the group consisting of ethano, propano and o-benzeno;
R⁴ is hydrogen, C₁-C₅ alkyl, C₁-C₅ hydroxyalkyl, C₆-C₁₂ aryl (which may be substituted with one or more substituents R¹⁷), NR¹⁰R¹¹, OR¹², COOR¹³ or CONR¹⁴R¹⁵, or R⁴ and R⁵ which substitutes on the peri-position cooperatively form R⁴-R⁵ that represent a bridging structure selected from the group consisting of N(R¹⁶)CO, N(R¹⁶)C(=NH), N(R¹⁶)CH₂, o-benzeno, ethano, propano and butano; R¹⁷ is fluoro, chloro, bromo, iodo, nitro, amino, hydroxy, C₁-C₅ alkyl, C₁-C₅ alkoxy, C₁-C₅ alkanoyloxy, trifluoromethyl, trifluoromethoxy or cyano; R¹⁰, R¹¹, R¹² and R¹⁶ independently are hydrogen, C₁-C₅ alkyl, C₄-C₇ cycloalkylalkyl, C₇-C₁₃ aralkyl or C₁-C₅ alkanoyl, and R¹³, R¹⁴ and R¹⁵ independently are hydrogen, C₁-C₅ alkyl, C₆-C₁₂ aryl or C₇-C₁₃ aralkyl)
or a pharmaceutically acceptable acid addition salt thereof.

By the present invention, novel therapeutic or prophylatic agent for frequent urination or urinary incontinence, of which side effects are reduced, was provided.

### Best Mode for Carrying Out the Invention

The present invention is a therapeutic or prophylatic agent for frequent urination or urinary incontinence, comprising as an effective ingredient the quinolinomorphinan derivatives represented by Formula (I) or a pharmaceutically acceptable acid addition salt thereof.

Among the quinolinomorphinan derivatives represented by Formula (I), those wherein R¹, R², R³, R⁵ and m represent the same meanings as described above, and R⁴ is hydrogen, C₁-C₅ alkyl, NR¹⁰R¹¹, or R⁴ and R⁵ which substitutes on the peri-position cooperatively form R⁴-R⁵ that represent a bridging structure of the formula N(R¹⁶)CO (wherein R¹⁰, R¹¹ and R¹⁶ represent the same meanings as described above) are preferred. Among these preferred derivatives, those wherein R¹ is C₁-C₅ alkyl, C₄-C₇ cycloalkylalkyl, C₇-C₁₃ aralkyl, furan-2-ylalkyl (wherein the number of carbon atoms in the alkyl moiety is 1 to 5) or thiophene-2-ylalkyl (wherein the number of carbon atoms in the alkyl moiety is 1 to 5) are especially preferred.

Each subsistent in the quinolinomorphinane derivatives represented by Formula (I) will now be described more concretely.

Preferred examples of R¹ are hydrogen, C₁-C₅ alkyl, C₄-C₇ cycloalkylmethyl, C₅-C₇ cycloalkenylmethyl, phenyl, naphthyl, C₇-C₁₃ phenylalkyl, C₂-C₇ alkenyl, C₁-C₅ alkanoyl, C₁-C₅ furan-2-ylalkyl (the number of carbon atoms mentioned here is the number of carbon atoms in the alkyl moiety in furan-2-ylalkyl) and C₁-C₅ thiophene-2-ylalkyl (the number of carbon atoms mentioned here is the number of carbon atoms in the alkyl moiety in thiophene-2-ylalkyl), and more preferred examples of R¹ are hydrogen, methyl, ethyl, propyl, butyl, cyclopropylmethyl, cyclobutylmethyl, cyclopentylmethyl, benzyl, phenethyl, phenylpropyl, allyl, 2-butenyl, 3-butenyl, 2-methyl-2-butenyl, 3-methyl-2-butenyl, 3-methyl-3-butenyl, acetyl, furan-2-ylmethyl, furan-2-ylethyl, furan-2-ylpropyl, thiophene-2-ylmethyl, thiophene-2-ylethyl and thiophene-2-ylpropyl. Among these, particularly preferred are hydrogen, methyl, ethyl, propyl, cyclopropylmethyl, phenethyl, furan-2-ylethyl and thiophene-2-ylethyl.

R² and R³ may preferably be hydrogen, hydroxy, methoxy, ethoxy, propoxy, acetoxy, benzyloxy or benzoyloxy. Among these, hydroxy, methoxy and acetoxy are preferred as R², and hydrogen, hydroxy and methoxy are preferred as R³.

R⁵ represents R¹⁸ when it does not form a fused ring structure A. In this case, R¹⁸ may preferably be fluoro, chloro, bromo, iodo, nitro, hydroxy, methyl, ethyl, propyl, butyl, methoxy, ethoxy, isothiocyanato, trifluoromethyl, trifluoromethoxy, cyano, phenyl, hydroxymethyl, SR⁶, SOR⁶, SO₂R⁶, CO₂R⁷, CH₂CO₂R⁷, (CH₂)₂CO₂R⁷, SO₂NR⁷R⁸, CONR⁷R⁸, NR⁷R⁸, CH₂NR⁷R⁸, (CH₂)₂NR⁷R⁸, N(R⁷)COR⁸, CH₂N(R⁷)COR⁸ or (CH₂)₂N(R⁷)COR⁸. Here, R⁶ may preferably be methyl or ethyl, and R⁷ may preferably be hydrogen or methyl, and R⁸ may preferably be hydrogen, methyl, ethyl, propyl, butyl, cyclopropylmethyl or cyclopropylbutyl. In cases where two R⁵s cooperatively form a fused ring structure A, the remaining 0 to 2 R⁵s represent the above-described R¹⁸, or the additional two R⁵s cooperatively form another fused ring structure A. As the fused ring structure A, preferred are benzo, indeno, naphtho, pyrido or cyclohexeno, each of which is substituted with 0 to 2 R⁹s, and non-substituted dioxoleno, and especially preferred are benzo, indeno or cyclohexeno, each of which is substituted with 0 or 1 R⁹, and non-substituted dioxoleno. For example, when two R⁵s cooperatively form one fused ring structure A, and this fused ring structure A is benzo, the compounds of Formula (I) may be exemplified by the following Formulae (IIa), (IIb),(IIc) and (IId), but these do not restrict Formula (I).

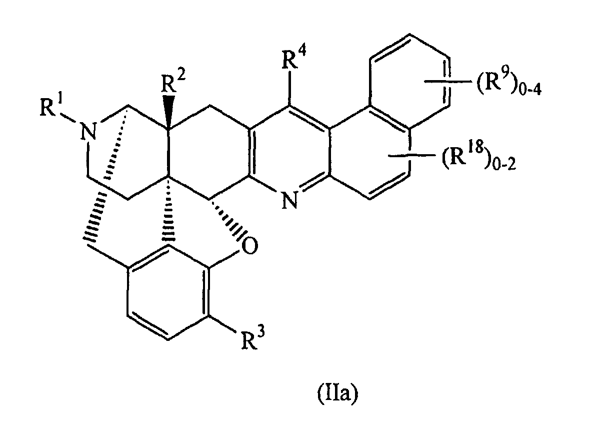

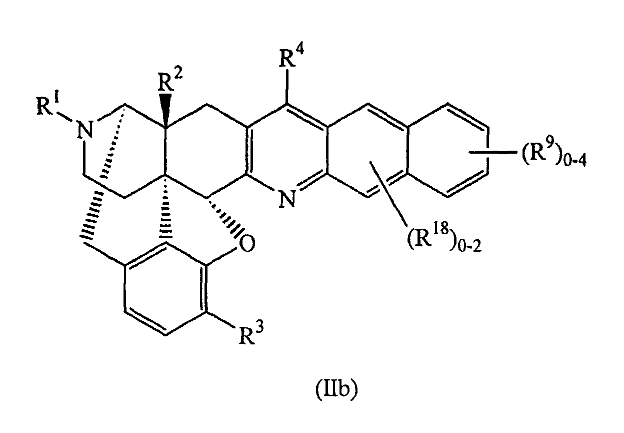

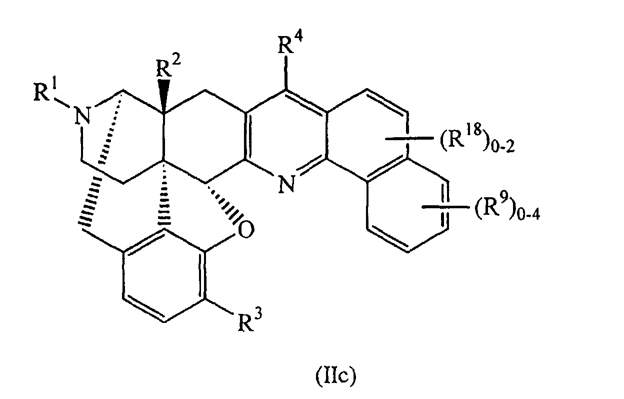

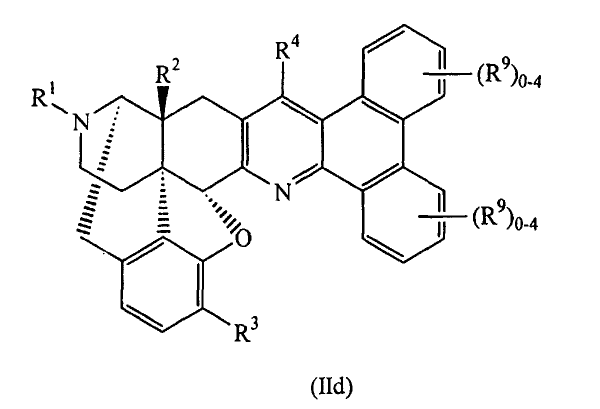

In cases where R⁹ does not form a bridging structure represented by R⁹-R¹⁸, preferred examples of R⁹ are fluoro, chloro, bromo, iodo, nitro, hydroxy, methyl, ethyl, propyl, butyl, methoxy, ethoxy, isothiocyanato, trifluoromethyl, trifluoromethoxy, cyano, phenyl, hydroxymethyl, SR⁶, SOR⁶, SO₂R⁶, CO₂R⁷, CH₂CO₂R⁷, (CH₂)₂CO₂R⁷, SO₂NR⁷R⁸, CONR⁷R⁸, NR⁷R⁸, CH₂NR⁷R⁸, (CH₂)₂NR⁷R⁸, N(R⁷)COR⁸, CH₂N(R⁷)COR⁸ and (CH₂)₂N(R⁷)COR⁸. Here, R⁶ may preferably be methyl or ethyl, R⁷ may preferably be hydrogen or methyl, and R⁸ may preferably be hydrogen, methyl, ethyl, propyl, butyl, cyclopropylmethyl or cyclopropylbutyl. R⁹ and R¹⁸ may cooperatively form a bridging structure R⁹-R¹⁸. In this case, as the bridging structure, ethano and o-benzeno are preferred. For example, when this bridging structure is ethano, and the above-mentioned fused ring structure A is benzo, the compounds of Formula (I) may be exemplified by the following Formulae (IIIa), (IIIb), (IIIc) and (IIId), but these do not restrict Formula (I).

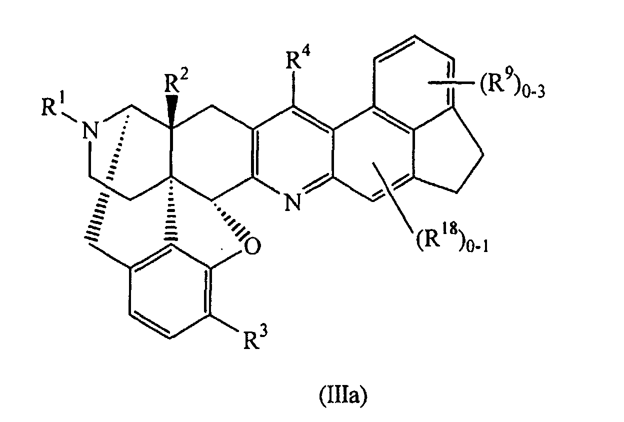

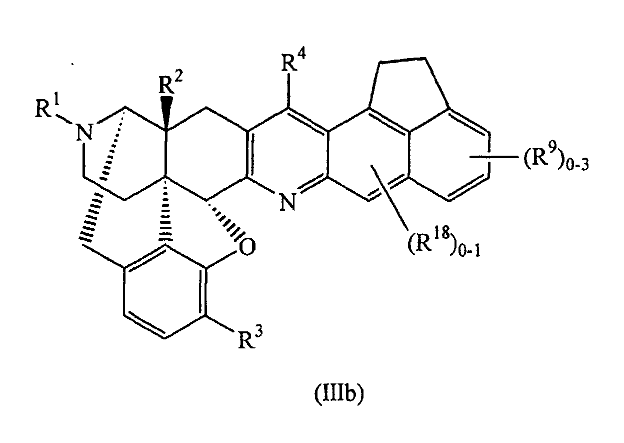

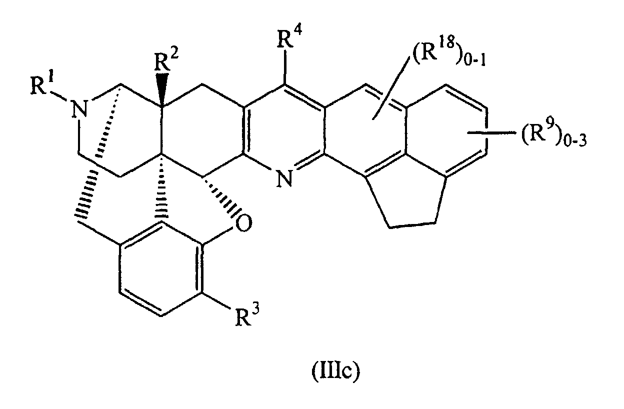

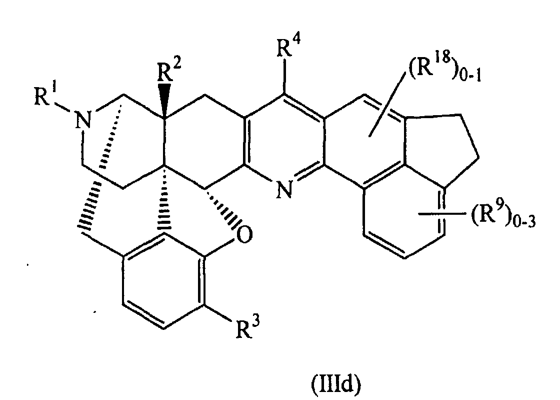

In cases where R⁴ and R⁵ which substitutes on the peri-position do not cooperatively form a bridging structure R⁴-R⁵, preferred examples of R⁴ are hydrogen, methyl, ethyl, propyl, isopropyl, C₁-C₃ hydroxyalkyl, C₆-C₁₂ aryl (which may be substituted with one or more substituents R¹⁷), NR¹⁰R¹¹, OR¹², COOR¹³ and CONR¹⁴R¹⁵. In these cases, R¹⁰ may preferably be hydrogen, methyl, ethyl, propyl or benzyl; R¹¹ may preferably be hydrogen, C₁-C₅ alkyl, C₄-C₇ cycloalkylalkyl, benzyl, phenethyl or C₁-C₅ alkanoyl; R¹² may preferably be hydrogen, methyl or acetyl; R¹³ may preferably be hydrogen, methyl, ethyl, phenyl or benzyl; R¹⁴ may preferably be hydrogen, methyl, ethyl, phenyl or benzyl; R¹⁵ may preferably be hydrogen, methyl, ethyl, phenyl or benzyl; and R¹⁷ may preferably be fluoro, chloro, bromo, amino, methyl or ethyl. Among the above-mentioned preferred examples of R⁴, more preferred are hydrogen, methyl, ethyl, isopropyl, hydroxymethyl, hydroxyethyl, phenyl, naphthyl, amino, methylamino, dimethylamino, (cyclohexylmethyl)amino, benzylamino, phenethylamino, formylamino, acetylamino, propionylamino, hydroxy, methoxy, acetoxy, carboxyl, methoxycarbonyl, ethoxycarbonyl, phenoxycarbonyl, benzyloxycarbonyl, carbamoyl, methylcarbamoyl, phenylcarbamoyl, benzylcarbamoyl and dimethylcarbamoyl. Among these, especially preferred are hydrogen, methyl, ethyl, isopropyl, hydroxymethyl, phenyl, amino, methylamino, dimethylamino, (cyclohexylmethyl)amino, benzylamino, formylamino, acetylamino, hydroxy, methoxy, carboxyl, methoxycarbonyl, carbamoyl, methylcarbamoyl and dimethylcarbamoyl. In cases where R⁴ and R⁵ which substitutes on the peri-position cooperatively form a bridging structure R⁴-R⁵, preferred examples of the bridging structure R⁴-R⁵ are N(R¹⁶)CO, N(R¹⁶)C(=NH), N(R¹⁶)CH₂, o-benzeno and propano, and especially preferred examples are N(R¹⁶)CO, N(R¹⁶)C(=NH) and N(R¹⁶)CH₂. In this case, R¹⁶ may preferably be hydrogen, methyl, ethyl, benzyl or acetyl. For example, R⁴ and R⁵ which substitutes on the peri-position cooperatively form a bridging structure R⁴-R⁵, and this bridging structure R⁴-R⁵ is N(R¹⁶)CO, the compounds of Formula (I) may be exemplified by the following Formulae (IV), but this does not restrict Formula (I).

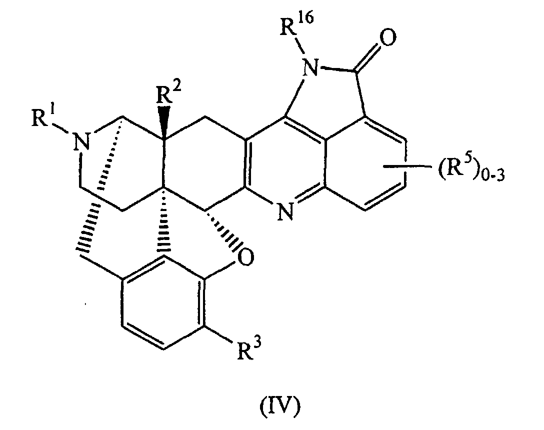

Preferred examples of the pharmaceutically acceptable acid addition salt include inorganic acid salts such as hydrochloric acid salt, sulfuric acid salt, nitric acid salt, hydrobromic acid salt, hydroiodic acid salt and phosphoric acid salt; organic carboxylic acid salts such as acetic acid salt, lactic acid salt, citric acid salt, oxalic acid salt, glutaric acid salt, malic acid salt, tartaric acid salt, fumaric acid salt, mandelic acid salt, maleic acid salt, benzoic acid salt and phthalic acid salt; and organic sulfonic acid salts such as methanesulfonic acid salt, ethanesulfonic acid salt, benzenesulfonic acid salt, p-toluenesulfonic acid salt and camphorsulfonic acid salt. Among these, hydrochloric acid salt, phosphoric acid salt, tartaric acid salt, methanesulfonic acid salt and the like are preferred, but the acid addition salt is not restricted thereto.

The compounds of Formula (I) may be produced by the method described in U.S. Patent 4,816,586, or International Publication WO98/43977.

The fact that the quinolinomorphinane derivatives represented by Formula (I) is effective for therapy or prophylaxis of urinary frequency or urinary incontinence may be confirmed by the effect of inhibiting rhythmic bladder contraction or by the effect of extending bladder reflex in animals. The effect of inhibiting rhythmic bladder contraction and the action of extending bladder reflex in animals may be carried out according to a reported method (Brain. Res., vol. 297, 191(1984) or J. Pharmcol. Exp. Ther., vol. 240, 978(1987)), but the method is not restricted thereto.

The compounds of the present invention may be used as pharmaceuticals useful for the therapy or prophylaxis of urinary frequency or urinary incontinence. Particularly, the compounds may be used for the therapy or prophylaxis of urinary frequency or urinary incontinence caused by the diseases such as neurogenic bladder, nocturia, overactive bladder, unstable bladder, pollakisuria nervosa, psycogenic frequency, enuresis, cystospasm, chronic cystitis, chronic prostatitis, prostatomegaly and prostate carcinoma. The term "neurogenic bladder" means that the function of urinary storage or evacuation of the lower urinary tract is in an abnormal state because of some damage of the nerve governing the lower urinary tract comprising bladder, urethra and external urinary sphincter. Examples of the diseases which damage the nerve include cerebrovascular disease, brain tumor, brain injury, encephalitis, normal pressure hydrocephalus, dementia, Parkinson's disease, striato-nigral degeneration, progressive supranuclear palsy, olivo-ponto-cerebellar atrophy, Shy-Drager syndrome, spinal cord injury, vascular disease of spinal cord, spinal cord tumor, myelitis, cervical cord compression disease, syringomyelia, multiple sclerosis, spina bifida, myelomeningocele, Tethered cord syndrome and myelopathy. However, use of the therapeutic or prophylatic agent for frequent urination or urinary incontinence according to the present invention is not restricted to these diseases.

When using the therapeutic or prophylatic agent for frequent urination or urinary incontinence according to the present invention as a pharmaceutical, the drug may be the free base or a salt thereof alone, or the drug may optionally be admixed with one or more additives such as vehicles, stabilizers, preservatives, buffering agents, solubilizers, emulsifiers, diluents and isotonic agents. The pharmaceutical may be administered either orally or parenterally. Examples of the parenteral administration include intravenous injection, subcutaneous injection, intramuscular injection and per rectum administration. The compound may be formulated into an injection solution, tablet, liquid, capsule, granule, powder or the like. The formulation may be carried out by known formulation techniques. Although the administration dose may be appropriately selected depending on the symptom, age and body weight of the patient, administration route and the like, the dose of the effective component per adult per day may be 0.0001 mg to 10 g, preferably 0.001 mg to 1 g, and may be administered in one time or dividedly in several times.

### Examples

The present invention will now be described more concretely by way of Reference Examples and Examples. The structure and the physical data of each of the compounds synthesized in the following Reference Examples are shown in Tables 1 to 8 below.

### Reference Example 1

### Synthesis of 17-cyclopropylmethyl-6,7-dehydro-4,5α-epoxy-3,14β-dihydroxy-6,7,2',3'-[4',5'-[imino(oxomethano)]quinolino]morphinan hydrochloric acid salt (Compound 1), 17-methyl-6,7-dehydro-4,5α-epoxy-3,14β-dihydroxy-6,7,2',3'-(4'-aminoquinolino)morphinan methanesulfonic acid salt (Compound 2) and 17-cyclopropylmethyl-6,7-dehydro-4,5α-epoxy-3,14β-dihydroxy-6,7,2',3'-(6',6"-ethano-7',8'-benzoquinolino)morphinan methanesulfonic acid salt (Compound 3)

The above-mentioned 3 compounds were synthesized according to the method described in International Publication WO98/43977.

### Reference Example 2

### Synthesis of 17-methyl-6,7-dehydro-4,5α-epoxy-3,14β-dihydroxy-6,7,2',3'-(4'-methylquinolino)morphinan methanesulfonic acid salt (Compound 4)

The compound was synthesized according to the method described in International Publication WO98/43977. In 6 mL of acetic acid, 315 mg (1.00 mmol) of 17-methyl-6-oxo-4,5α-epoxy-14β-hydroxy-3-methoxy-morphinan and 133 µL (1.10 mmol) of o-aminoacetophenone were dissolved, and the mixture was heated to reflux, thereby carrying out synthesis of quinoline. After concentrating the reaction solution under reduced pressure, saturated aqueous sodium hydrogen carbonate solution was added to the obtained residue, and the resulting mixture was extracted with ethyl acetate. Organic layers were combined, dried over anhydrous sodium sulfate, and concentrated to obtain a crude product, followed by purification of the crude product by silica gel column chromatography to obtain 382 mg (yield 92%) of 17-methyl-6,7-dehydro-4,5α-epoxy-14β-hydroxy-3-methoxy-6,7,2',3'-(4'-methylquinolino)morphinan. This purified product was dissolved in 10 mL of DMF, and then 0.40mL (4.58 mmol) of *n*-propanethiol and 496 mg (4.42 mmol) of potassium *t*-butoxide were added thereto, followed by allowing the mixture to react at 120 °C for 3 hours. After cooling the reaction solution, saturated aqueous sodium hydrogen carbonate solution was added and the resulting mixture was extracted with chloroform:methanol (4:1) mixed solvent. Organic layers were combined, dried over anhydrous sodium sulfate and concentrated to obtain a crude product which was then purified by silica gel column chromatography to obtain 181 mg (yield 49%) of the captioned compound in free form. This was converted to methanesulfonic acid salt to obtain the captioned Compound 4.

### Reference Example 3

### Synthesis of 17-methyl-6,7-dehydro-4,5α-epoxy-3,14β-dihydroxy-6,7,2',3'-(4'-hydroxyquinolino)morphinan methanesulfonic acid salt (Compound 5)

In a mixed solvent of 2.5 mL of acetic acid and 4 mL of 20% aqueous sulfuric acid solution, 400 mg (0.96 mmol) of 17-methyl-6,7-dehydro-4,5α-epoxy-14β-dihydroxy-3-methoxy-6,7,2',3'-(4'-aminoquinolino)morphinan synthesized by the method described in Reference Example 1 was dissolved, and the mixture was cooled to 0 °C. To this mixture, 87 mg of sodium nitrite as 1 mL of aqueous solution was added dropwise, and the resulting mixture was stirred at 0 °C for 1 hour, followed by heating the resulting mixture to reflux for 4 hours. After allowing the reaction solution to cool, 2 N aqueous sodium hydroxide solution was added to make the mixture basic, and the resulting mixture was extracted with chloroform:methanol (4:1) mixed solvent. Organic layers were combined, dried over anhydrous sodium sulfate and concentrated to obtain a crude product which was then purified by silica gel column chromatography to obtain 167 mg (yield 40%) of 17-methyl-6,7-dehydro-4,5α-epoxy-14β-hydroxy-3-methoxy-6,7,2',3'-(4'-hydroxyquinolino)morphinan. This purified product was dissolved in 7 mL of DMF, and then 0.17 mL (1.84 mmol) of *n*-propanethiol and 215 mg (1.92 mmol) of potassium *t*-butoxide were added, followed by allowing the resulting mixture to react at 120 °C for 3 hours. After allowing the reaction solution to cool, saturated aqueous sodium hydrogen carbonate solution was added, and the resulting mixture was extracted with chloroform:methanol (4:1) mixed solvent. Organic layers were combined, dried over anhydrous sodium sulfate and concentrated to obtain a crude product, which was then purified by silica gel column chromatography to obtain 69 mg (yield 45%) of the captioned compound in free form. This was converted to methanesulfonic acid salt to obtain the captioned Compound 5.

### Reference Example 4

### Synthesis of 17-methyl-6,7-dehydro-4,5α-epoxy-3,14β-dihydroxy-6,7,2',3'-quinolinomorphinane methanesulfonic acid salt (Compound 6)

In accordance with the method described in Reference Example 2, the captioned Compound 6 was obtained by using *o*-aminobenzaldehyde in place of *o*-aminoacetophenone.

### Reference Example 5

### Synthesis of 17-phenethyl-6,7-dehydro-4,5 α-epoxy-3,14β-dihydroxy-6,7,2',3'-quinolinomorphinan methanesulfonic acid salt (Compound 7)

To 150 mg (0.39 mmol) of 17-hydrogen-6,7-dehydro-4,5α-epoxy-14β-dihydroxy-3-methoxy-6,7,2',3'-quinolinomorphinan, were added 240 mg (0.80 mmol) of phenylacetoaldehyde and 165 mg (0.78 mmol) of sodium borotriacetoxy hydride, and the resulting mixture was dissolved in 5 mL of 1,2-dichloroethane and 22 µL of acetic acid, followed by stirring the resulting mixture at room temperature for 2 hours. After the reaction compreted, saturated aqueous sodium hydrogen carbonate solution was added and the resulting solution was extracted with chloroform. Organic layers were combined, dried over anhydrous sodium sulfate and concentrated to obtain a crude product which was then purified by silica gel column chromatography to obtain 186 mg (yield 97%) of 17-phenethyl-6,7-dehydro-4,5α-epoxy-14β-hydroxy-3-methoxy-6,7,2',3'-quinolinomorphinan. This purified product was dissolved in 5 mL of DMF, and then 0.19 mL (2.10 mmol) of *n*-propanethiol and 210 mg (1.87 mmol) of potassium *t*-butoxide were added, followed by allowing the resulting mixture to react at 120 °C for 3 hours. After allowing the reaction solution to cool, saturated aqueous sodium hydrogen carbonate solution was added, and the resulting mixture was extracted with chloroform:methanol (4:1) mixed solvent. Organic layers were combined, dried over anhydrous sodium sulfate and concentrated to obtain a crude product, which was then purified by silica gel column chromatography to obtain 143 mg (yield 79%) of the captioned compound in free form. This was converted to methanesulfonic acid salt to obtain the captioned Compound 7.

### Reference Example 6

### Synthesis of 17-(2-thienyl)ethyl-6,7-dehydro-4,5α-epoxy-3,14β-dihydroxy-6,7,2',3'-quinolinomorphinan methanesulfonic acid salt (Compound 8)

In 8 mL of benzene, 99 mg (0.39 mmol) of 17-hydrogen-6,7-dehydro-4,5α-epoxy-14β-dihydroxy-3-methoxy-6,7,2',3'-morphinan synthesized by the method described in Reference Example 1 was dissolved, and 327 mg (1.16 mmol) of (2-thienyl)ethyltosylate and 0.36 mL (2.58 mmol) of triethylamine were added, followed by heating the mixture to reflux overnight under stirring. After allowing the reaction solution to cool, saturated aqueous sodium hydrogen carbonate solution was added and the mixture was extracted with chloroform. Organic layers were combined, dried over anhydrous sodium sulfate and concentrated to obtain a crude product which was then purified by silica gel column chromatography to obtain 112 mg (yield 88%) of 17-(2-thienyl)ethyl-6,7-dehydro-4,5α-epoxy-14β-hydroxy-3-methoxy-6,7,2',3'-quinolinomorphinan. The obtained purified product was dissolved in 5 ml of DMF, and then 0.10 mL (1.10 mmol) of *n*-propanethiol and 124 mg (1.11 mmol) of potassium *t*-butoxide were added, followed by allowing the mixture to react at 120 °C for 3 hours. After cooling the reaction solution, saturated aqueous sodium hydrogen carbonate solution was added and the resulting mixture was extracted with chloroform:methanol (4:1) mixed solvent. Organic layers were combined, dried over anhydrous sodium sulfate and concentrated to obtain a crude product, which was then purified by silica gel column chromatography to obtain 85 mg (yield 79%) of the captioned compound in free form. This was converted to methanesulfonic acid salt to obtain the captioned Compound 8.

### Reference Example 7

### Synthesis of 17-methyl-6,7-dehydro-4,5 α-epoxy-3-hydroxy-14 β -methoxy-6,7,2',3'-quinolinomorphinan methanesulfonic acid salt (Compound 9)

In 7 mL of dimethylformamide, 200 mg (0.50 mmol) of the synthetic intermediate 17-methyl-6,7-dehydro-4,5α-epoxy-14β-hydroxy-3-methoxy-6,7,2',3'-quinolinomorphinane described in Reference Example 4 was dissolved, and the mixture was cooled to 0°C. To the mixture, 24 mg (1.00 mmol) of sodium hydride was added and the resulting mixture was stirred for 15 minutes. Then, 47 µl (0.75 mmol) of iodomethane was added and the reaction mixture was stirred for 1 hour. Saturated aqueous sodium hydrogen carbonate solution was added to the reaction mixture, and the resulting mixture was extracted with ethyl acetate. Organic layers were combined, dried over anhydrous sodium sulfate and concentrated to obtain a crude product which was then purified by silica gel column chromatography to obtain 166 mg (yield 80%) of 17-methyl-6,7-dehydro-4,5α-epoxy-3,14β-dimethoxy-6,7,2',3'-quinolinomorphinan. This purified product was dissolved in 10 mL of methylene chloride, and the mixture was cooled to -20 °C. To the mixture, 1.9 mL (1.93 mmol) of 1N boron tribromide solution was added and the resulting mixture was stirred for 1 hour. To the obtained mixture, 20 mL of 20% aqueous ammonia solution was added and the resulting mixture was stirred at room temperature for 30 minutes, followed by extraction of the resulting mixture with chloroform:methanol (4:1) mixed solvent. Organic layers were combined, dried over anhydrous sodium sulfate and concentrated to obtain a crude product, which was then purified by silica gel column chromatography to obtain 99 mg (yield 64%) of the captioned compound in free form. This was converted to methanesulfonic acid salt to obtain the captioned Compound 9.

### Reference Example 8

### Synthesis of 17-methyl-6,7-dehydro-4,5α-epoxy-3,14β-dihydroxy-6,7,2',3'-(8'-chloroquinolino)morphinan methanesulfonic acid salt (Compound 10)

To 500 mg (1.35 mmol) of 17-methyl-6-oxo-4,5α-epoxy-14β-hydroxy-3-methoxymorphinan and 0.58 mL (5.40 mmol) of 2-chloroaniline, 10 mL of trifluoroacetic acid was added and the mixture was heated to reflux. The reaction solution was concentrated under reduced pressure to obtain a residue, and aqueous sodium hydrogen carbonate solution was added to the residue, followed by extraction of the resulting mixture with ethyl acetate. Organic layers were combined, dried over anhydrous sodium sulfate and concentrated to obtain a crude product, which was then purified by silica gel column chromatography to obtain a mixture of 17-methyl-6,7-dehydro-4,5α-epoxy-14β-hydroxy-3-methoxy-6,7,2',3'-(8'-chloroquinolino)morphinan and non-cyclized compound. This mixture was dissolved in 10 mL of THF, and 10 mL of 6 N hydrochloric acid was added, followed by heating the resulting mixture to reflux. The reaction solution was concentrated under reduced pressure to obtain a residue, and aqueous sodium hydrogen carbonate solution was added to the residue, followed by extraction of the resulting mixture with ethyl acetate. Organic layers were combined, dried over anhydrous sodium sulfate and concentrated to obtain a crude product, which was then purified by silica gel column chromatography to obtain 290 mg (yield 49%) of 17-methyl-6,7-dehydro-4,5α-epoxy-14β-hydroxy-3-methoxy-6,7,2',3'-(8'-chloroquinolino)morphinan. After dissolving this purified product in 10 mL of dichloromethane, 2.0 mL of 1 N boron tribromide solution was added thereto at 0 °C and the mixture was stirred for 15 minutes, and then at room temperature for 30 minutes. After cooling the reaction solution to 0 °C, 10 mL of 7% aqueous ammonia solution was added and the mixture was stirred for 1 hour, followed by extraction of the reaction solution with chloroform. Organic layers were combined, dried over anhydrous sodium sulfate and concentrated to obtain a crude product, which was then purified by silica gel column chromatography to obtain 139 mg (yield 50%) of the captioned compound in free form. This was converted to methanesulfonic acid salt to obtain the captioned Compound 10.

### Reference Example 9

### Synthesis of 17-methyl-6,7-dehydro-4,5α-epoxy-3,14β-dihydroxy-6,7,2',3'-(6'-chloroquinolino)morphinan methanesulfonic acid salt (Compound 11)

According to the method described in Reference Example 8, by using 4-chloroaniline in place of 2-chloroaniline, and by using xylene as the solvent, a mixture of 17-methyl-6,7-dehydro-4,5α-epoxy-14β-hydroxy-3-methoxy-6,7,2',3'-(6'-chloroquinolino)morphinan and a non-cyclized compound was obtained. After dissolving this mixture in 12 mL of methanol, 220 mg (5.80 mmol) of sodium borohydride was added thereto at 0 °C and the mixture was stirred for 15 minutes, and then at room temperature for 16 hours. Saturated aqueous sodium hydrogen carbonate solution was added to the reaction solution and the mixture was concentrated under reduced pressure, followed by extraction of the obtained residue with ethyl acetate. Organic layers were combined, dried over anhydrous magnesium sulfate and concentrated to obtain a crude product, which was then purified by silica gel column chromatography to obtain 163 mg (yield 38%) of 17-methyl-6,7-dehydro-4,5α-epoxy-14β-hydroxy-3-methoxy-6,7,2',3'-(6'-chloroquinolino)morphinan. This was converted to methanesulfonic acid salt to obtain the captioned Compound 11.

### Reference Example 10

### Synthesis of 17-methyl-6,7-dehydro-4,5α-epoxy-3,14β-dihydroxy-6,7,2',3'-(5'-chloroquinolino)morphinan tartaric acid salt (Compound 12)

According to the method described in Reference Example 9, by using 3-chloroaniline in place of 4-chloroaniline, free form of the captioned compound was obtained. This was converted to tartaric acid salt to obtain the captioned Compound 12.

### Reference Example 11

### Synthesis of 17-methyl-6,7-dehydro-4,5α-epoxy-3,14β-dihydroxy-6,7,2',3'-(8'-methylquinolino)morphinan methanesulfonic acid salt (Compound 13)

According to the method described in Reference Example 9, by using *o*-toluidine in place of 4-chloroaniline, the captioned Compound 13 was obtained.

### Reference Example 12

### Synthesis of 17-methyl-6,7-dehydro-4,5α-epoxy-3,14β-dihydroxy-6,7,2',3'-(6'-methylquinolino)morphinan methanesulfonic acid salt (Compound 14)

According to the method described in Reference Example 9, by using *p*-toluidine in place of 4-chloroaniline, the captioned Compound 14 was obtained.

### Reference Example 13

### Synthesis of 17-methyl-6,7-dehydro-4,5α-epoxy-3,14β-dihydroxy-6,7,2',3'-(8'-fluoroquinolino)morphinan tartaric acid salt (Compound 15)

According to the method described in Reference Example 10, by using 2-fluoroaniline in place of 3-chloroaniline, the captioned Compound 15 was obtained.

### Reference Example 14

### Synthesis of 17-methyl-6,7-dehydro-4,5α-epoxy-3,14β-diacetoxy-6,7,2',3'-quinolinomorphinan tartaric acid salt (Compound 16)

In 5 mL of pyridine, 200 mg (0.39 mmol) of 17-methyl-6,7-dehydro-4,5α-epoxy-3,14β-hydroxy-6,7,2',3'-quinolinomorphinan synthesized by the method described in Reference Example 4 was dissolved, and 10 mL (1.10 mmol) of acetic anhydride was added, followed by stirring the mixture at room temperature for 16 hours. The reaction solution was concentrated under reduced pressure and saturated aqueous sodium hydrogen carbonate solution was added to the obtained residue, followed by extraction of the resulting mixture with chloroform. Organic layers were combined, dried over anhydrous magnesium sulfate and concentrated to obtain a crude product, which was then purified by silica gel column chromatography to obtain 250 mg (yield 98%) of the captioned compound in free form. This was converted to tartaric acid salt to obtain the captioned Compound 16.

### Reference Example 15

Synthesis of 17-methyl-6,7-dehydro-4,5α-epoxy-3-hydroxy-14β-acetoxy-6,7,2',3'-quinolinomorphinan tartaric acid salt (Compound 17)

In a mixed solvent of 10 mL of methanol and 2 mL of chloroform, 156 mg (0.03 mmol) of 17-methyl-6,7-dehydro-4,5α-epoxy-3,14β-diacetoxy-6,7,2',3'-quinolinomorphinan synthesized by the method described in Reference Example 14 was dissolved, and 68.1 mg (0.49 mmol) of potassium carbonate was added, followed by stirring the resulting mixture at room temperature for 1 hour. Saturated aqueous sodium hydrogen carbonate solution was added to the reaction solution, and the mixture was concentrated under reduced pressure, followed by extraction of the obtained residue with chloroform. Organic layers were combined, dried over anhydrous magnesium sulfate and concentrated to obtain a crude product, which was then purified by silica gel column chromatography to obtain 104 mg (yield 73%) of the captioned compound in free form. This was converted to tartaric acid salt to obtain the captioned Compound 17.

### Reference Example 16

### Synthesis of 17-methyl-6,7-dehydro-4,5α-epoxy-3 -acetoxy-14β-hydroxy-6,7,2',3'-quinolinomorphinane tartaric acid salt (Compound 18)

In 5 mL of pyridine, 150 mg (0.39 mmol) of 17-methyl-6,7-dehydro-4,5α-epoxy-3,14β-dihydroxy-6,7,2',3'-quinolinomorphinan synthesized by the method described in Reference Example 4 and 109 µL (1.16 mmol) of acetic anhydride were dissolved, and the mixture was stirred at room temperature for 3 hours. The reaction solution was twice or thrice subjected to azeotropic distillation with toluene, and the mixture was concentrated to obtain a crude product, which was then purified by silica gel chromatography to obtain 110 mg (yield 66%) of 17-methyl-6,7-dehydro-4,5α-epoxy-3-acetoxy-14β-hydroxy-6,7,2',3'-quinolinomorpholin. This was converted to tartaric acid salt to obtain the captioned Compound 18.

### Reference Example 17

### Synthesis of 17-methyl-6,7-dehydro-4,5α-epoxy-3-methoxy-14β-hydroxy-6,7,2',3'-quinolinomorphinan tartaric acid salt (Compound 19)

17-methyl-6,7-dehydro-4,5α-epoxy-14β-hydroxy-3-methoxy-6,7,2',3'-quinolinomorphinan synthesized by the method of Reference Example 4 was converted to tartaric acid salt to obtain the captioned Compound 19.

### Reference Example 18

### Synthesis of 17-methyl-6,7-dehydro-4,5α-epoxy-14β-hydroxy-6,7,2',3'-quinolinomorphinan tartaric acid salt (Compound 20)

In 10 mL of ethanol, 112 mg (0.39 mmol) of 17-methyl-6-oxo-4,5α-epoxy-14β-hydroxy-morphinan, 143 mg (1.18 mmol) of *o*-aminobenzaldehyde and 77 µL (11.8 mmol) of methanesulfonic acid were dissolved, and the mixture was heated to reflux for 3 hours, thereby carrying out quinoline synthesis. The reaction solution was concentrated under reduced pressure to obtain a residue, and saturated aqueous sodium hydoren carbonate solution was added to the residue, followed by extraction of the resulting mixture with ethyl acetate. Organic layers were combined, dried over anhydrous sodium sulfate and concentrated to obtain a crude product, which was then purified by silica gel column chromatography to obtain 114 mg (yield 79%) of the captioned compound in free form. This was converted to tartaric acid salt to obtain the captioned Compound 20.

### Reference Example 19

### Synthesis of 17-cyclopropylmethyl-6,7-dehydro-4,5α-epoxy-3,14β-dihydroxy-6,7,2',3'-quinolinomorphinan methanesulfonic acid salt (Compound 21)

The compound was synthesized by the method described in U.S. Patent 4,816,586.

### Reference Example 20

### Synthesis of 17-allyl-6,7-dehydro-4,5α-epoxy-3,14β-dihydroxy-6,7,2',3'-quinolinomorphinan methanesulfonic acid salt (Compound 22)

The compound was synthesized by the method described in U.S. Patent 4,816,586.

### Reference Example 21

### Synthesis of 17-(4-methylphenyl)ethyl-6,7-dehydro-4,5α-epoxy-3,14β-dihydroxy-6,7,2',3'-quinolinomorphinan methanesulfonic acid salt (Compound 23)

In 8 mL of benzene, 95 mg (0.25 mmol) of 17-hydrogen-6,7-dehydro-4,5α-epoxy-14β-hydroxy-3-methoxy-6,7,2',3'-morphinan synthesized by the method described in Reference Example 1 was dissolved, and 335 mg (1.15 mmol) of (4-methylphenyl)ethyl tosylate and 0.30 mL (2.15 mmol) of triethylamine were added, followed by heating the resulting mixture to reflux overnight under stirring. After allowing the reaction solution to cool, saturated aqueous sodium hydrogen carbonate solution was added and the resulting mixture was extracted with chloroform. Organic layers were combined, dried over anhydrous sodium sulfate and concentrated to obtain a crude product, which was then purified by silica gel column chromatography to obtain 90 mg (yield 73%) of 17-(4-methylphenyl)ethyl-6,7-dehydro-4,5α-epoxy-14β-hydroxy-3-methoxy-6,7,2',3'-quinolinomorphinan.

This purified product was dissolved in 5 ml of dichloromethane and the mixture was cooled to 0 °C. Thereafter, 1.30 mL (1.30 mmol) of 1 N boron tribromide solution was added, and then the mixture was stirred at room temperature for 30 minutes. After adding 6 ml of aqueous ammonia solution, the reaction mixture was stirred for an additional 1 hour. Saturated aqueous sodium hydrogen carbonate solution was added to the reaction mixture and the resulting mixture was extracted with chloroform. Combined organic layers were washed with brine and dried over magnesium sulfate, followed by concentration of the resultant under reduced pressure. The obtained crude product was purified by silica gel column chromatography to obtain 83 mg (yield 95%) of 17-(4-methylphenyl)ethyl-6,7-dehydro-4,5α-epoxy-3,14β-dihydroxy-6,7,2',3'-quinolinomorphinan. This was converted to methanesulfonic acid salt to obtain the captioned Compound 23.

### Reference Example 22

### Synthesis of 17-(4-fluorophenyl)ethyl-6,7-dehydro-4,5α-epoxy-3,14β-dihydroxy-6,7,2',3'-quinolinomorphinan methanesulfonic acid salt (Compound 24)

According to the method described in Reference Example 21, by using (4-fluorophenyl)ethyl tosylate in place of (4-methylphenyl)ethyl tosylate, the captioned Compound 24 was obtained.

The chemical structure, acid addition salts and various spectra data of the above-described Compounds 1 to 24 are shown in the following tables.

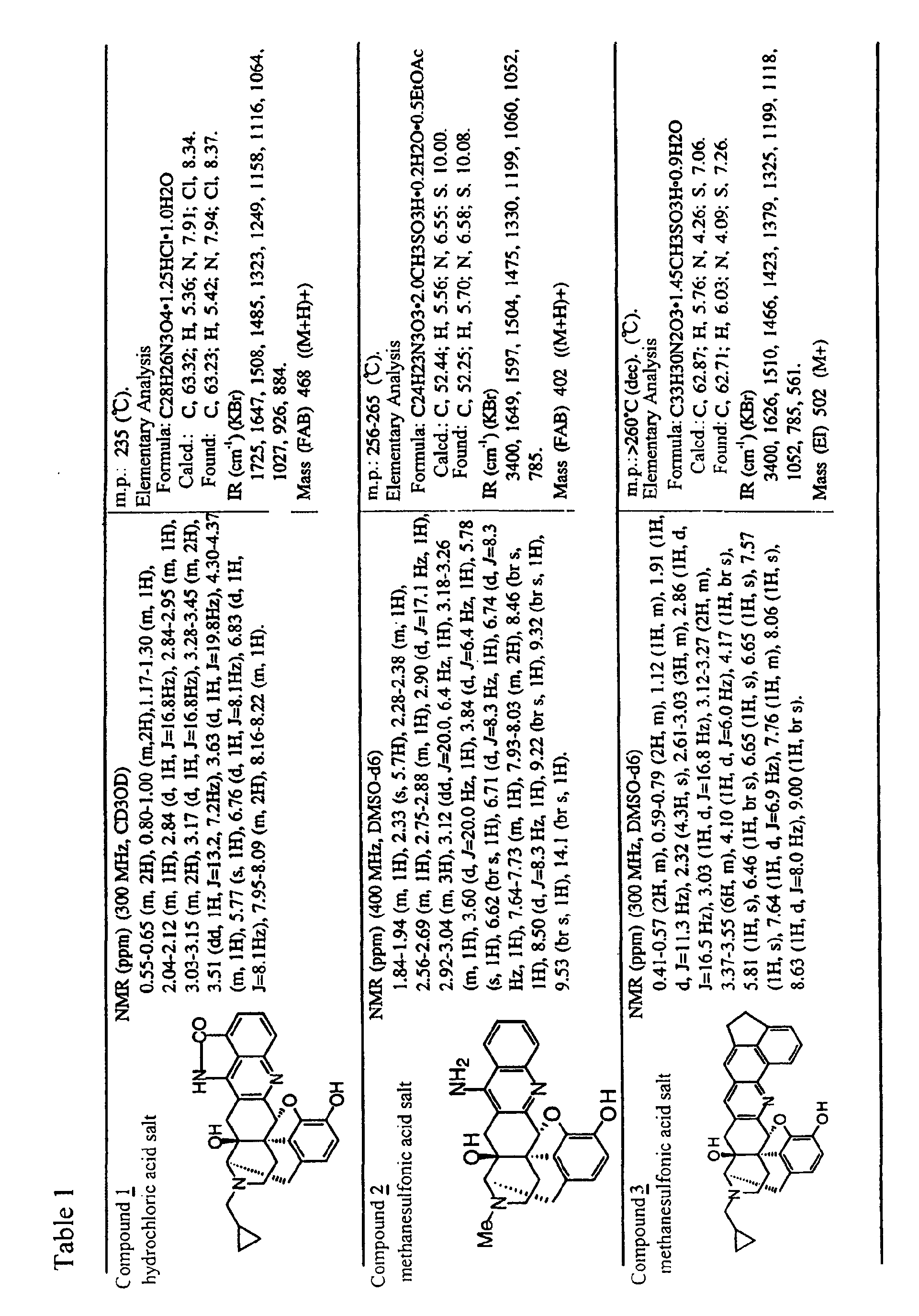

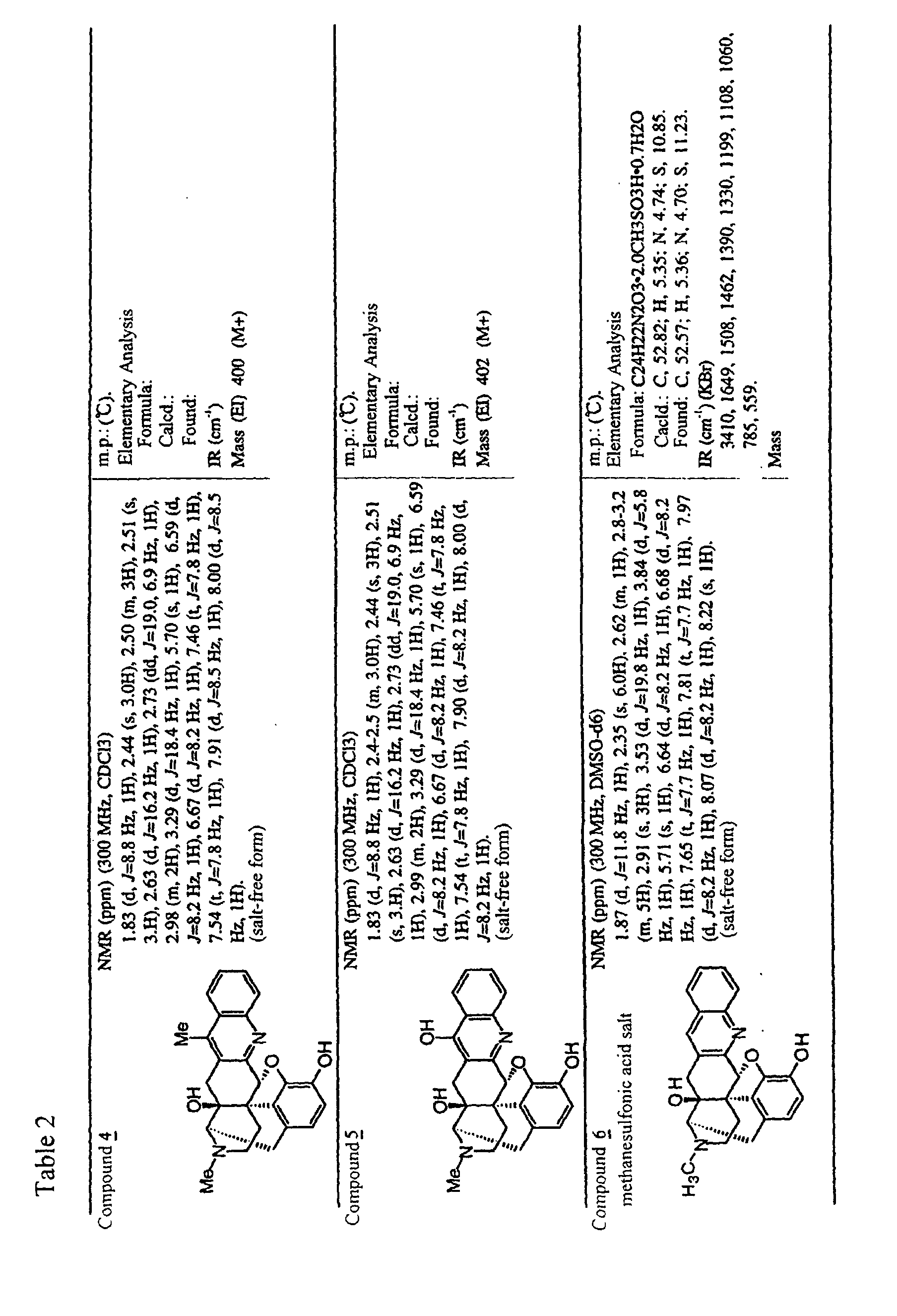

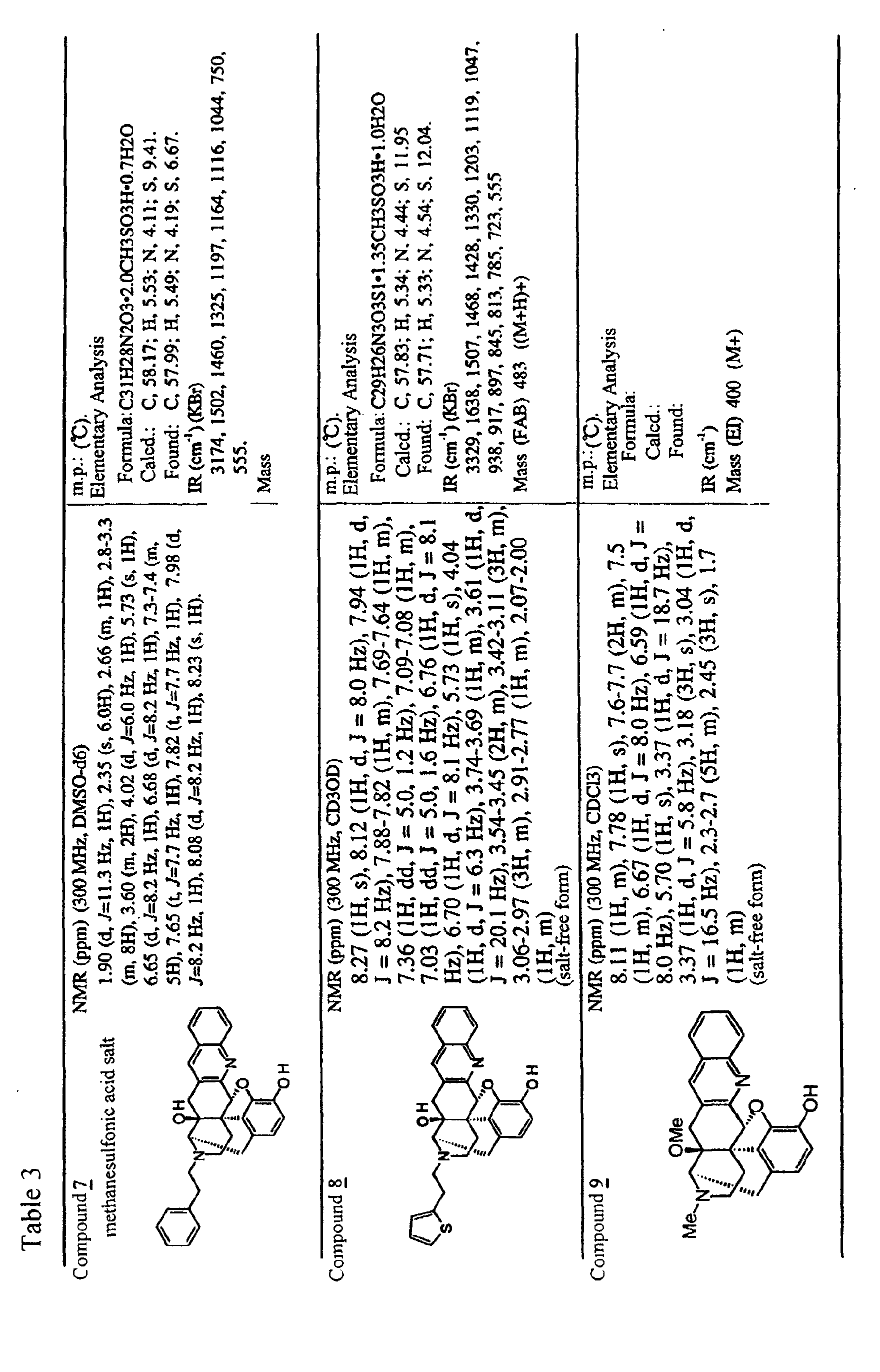

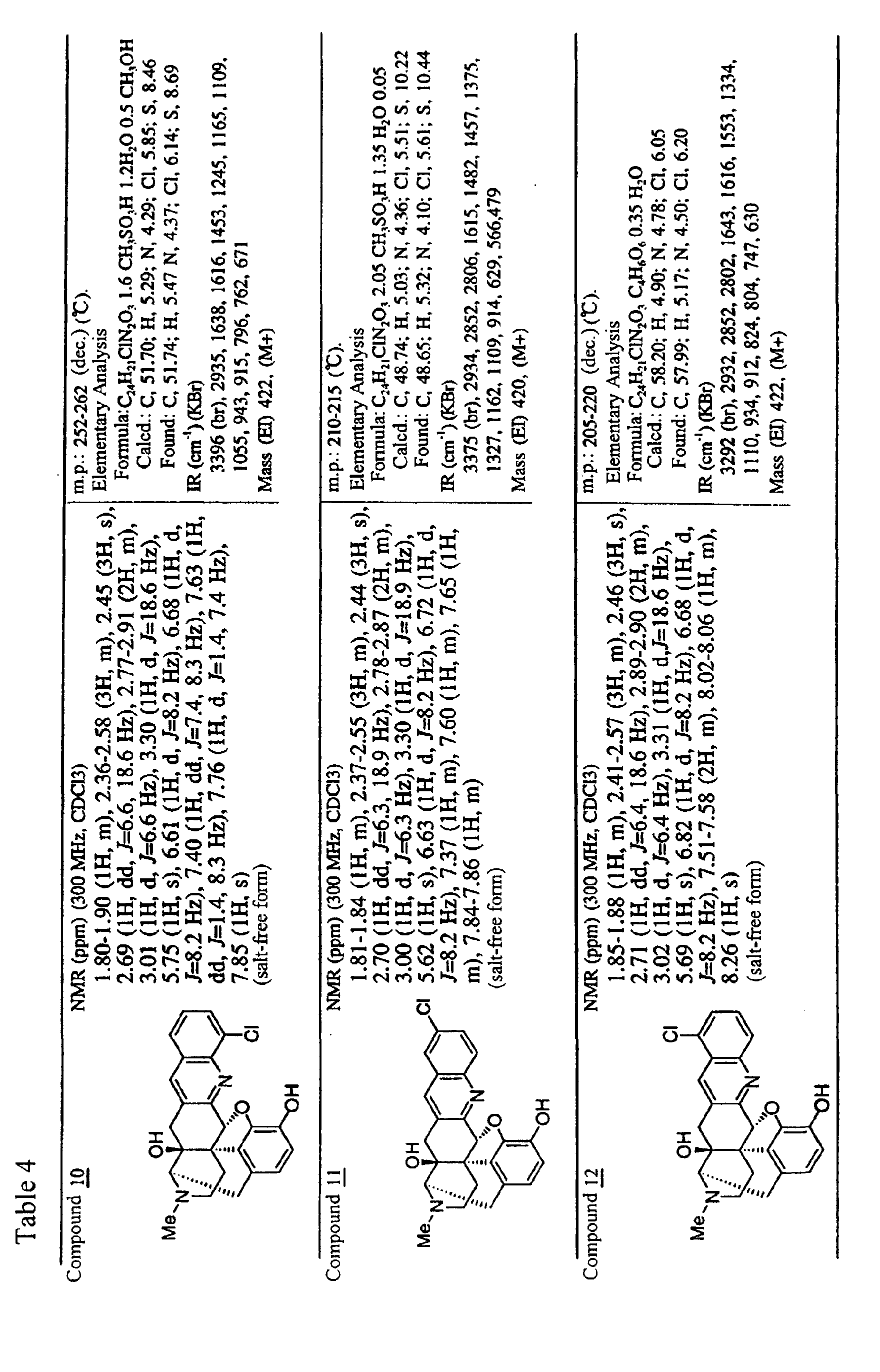

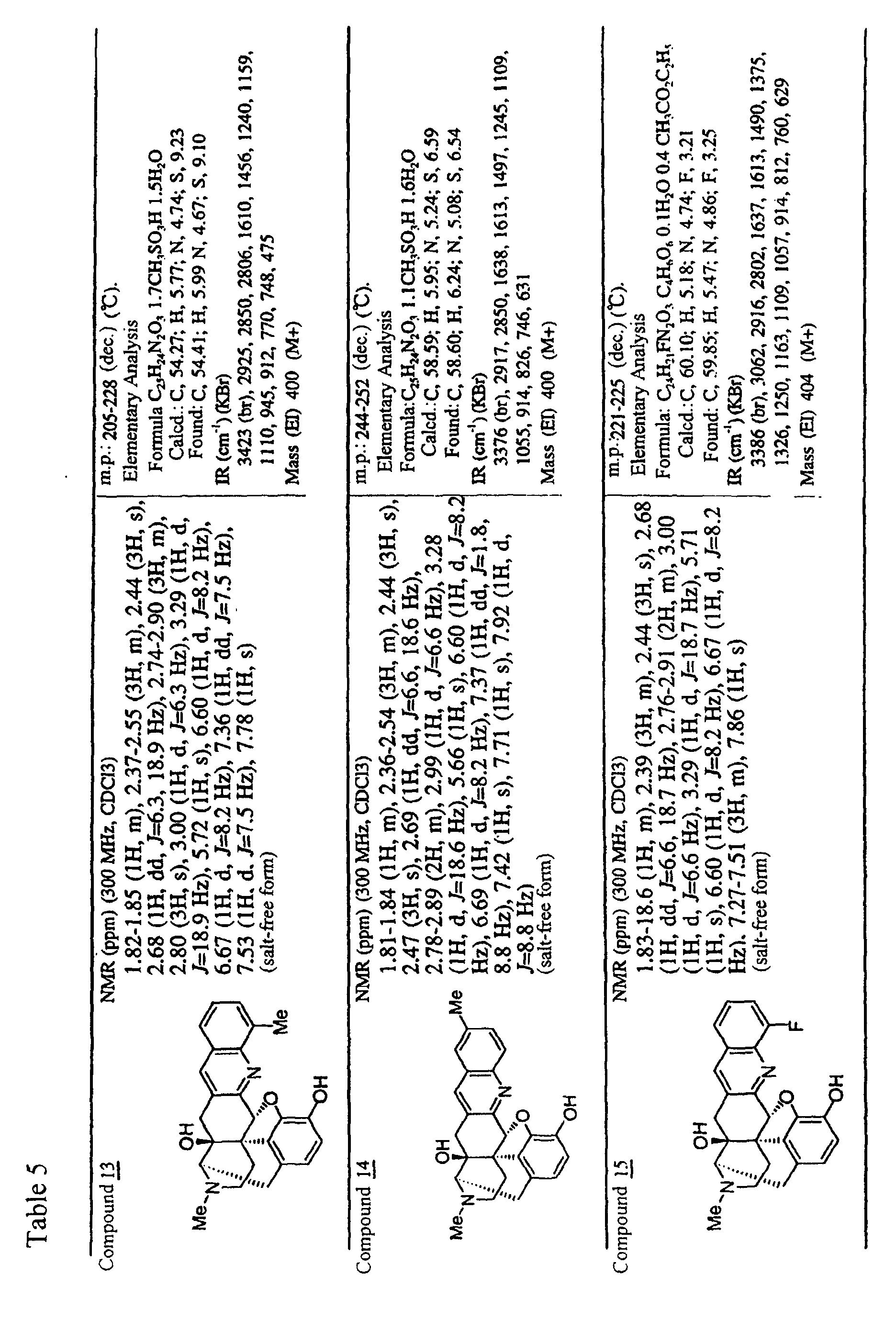

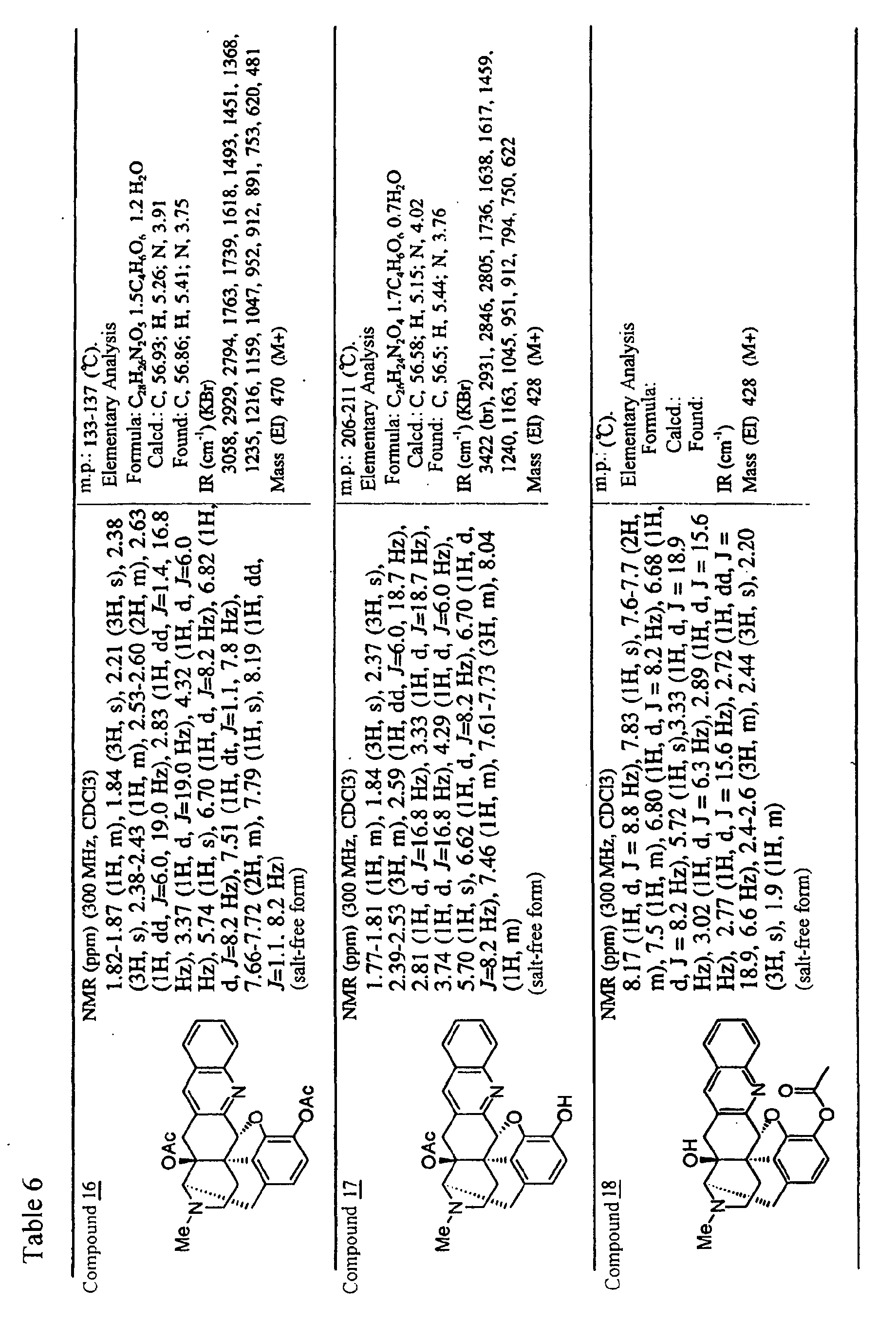

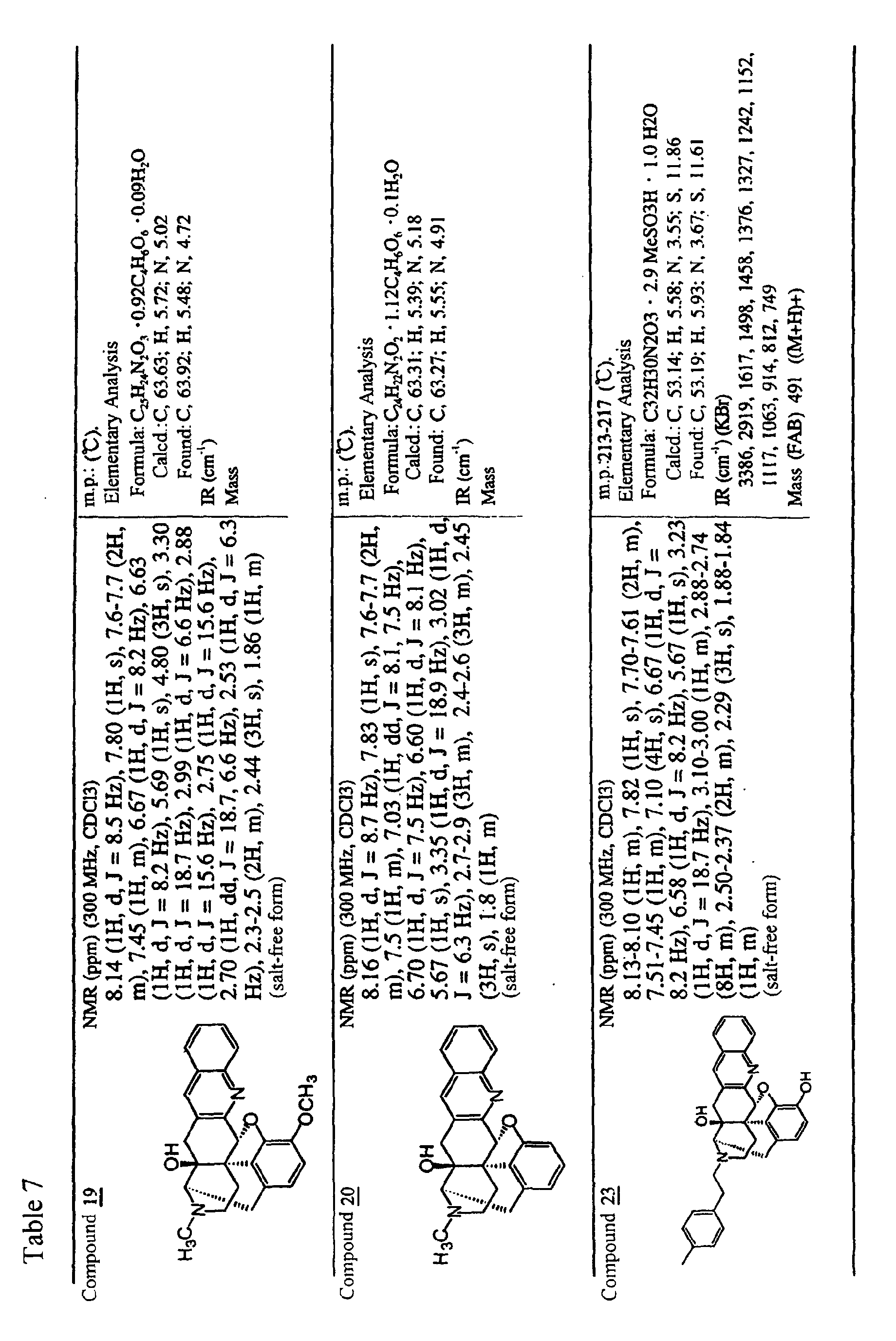

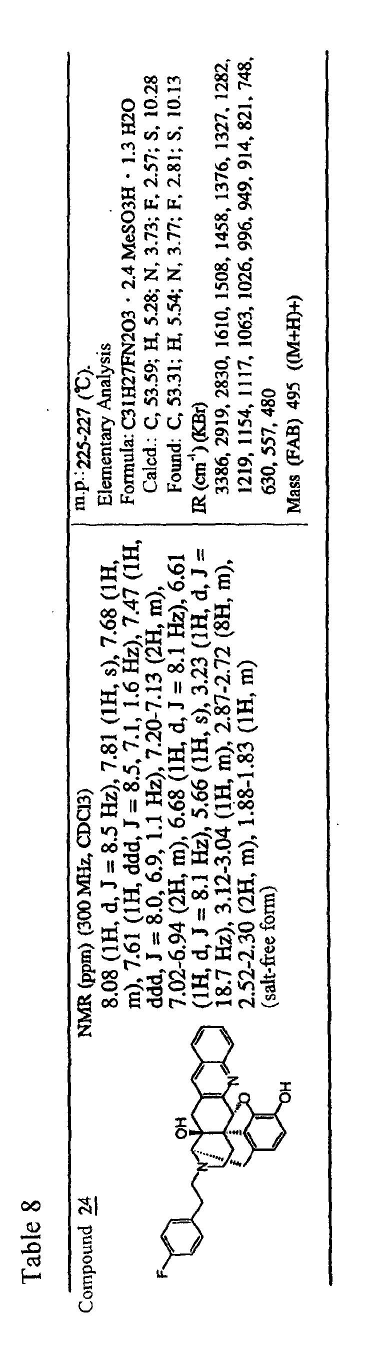

### Example 1

### Effect on Rhythmic Bladder Contraction of Rats

Female SD rats were anesthetized with an intraperitoneal injection of urethane (1.0 g/kg). A polyethylene tube was inserted from urethral opening to the bladder and the tube was fixed by ligating. Physiological saline was appropriately infused via the tube (rate of infusion: about 0.2 ml/min; maximum dose: about 1.5 ml/rat) to induce rhythmic contraction of bladder. The rhythmic contraction of bladder was monitored by measuring the intravesical pressure through the polyethylene tube inserted into the bladder. After confirming that stable rhythmic contraction occurred at least 10 times, each test compound was intravenously administered at a doseof 1 ml/kg. If the intravesical pressure became, within 10 minutes from the administration of the test compound, not more than 50% of the intravesical pressure at the time point immediately after the administration of the test compound, it was judged that bladder contraction occurred, and the time period until the intravesical pressure again became not less than 50% was defined as the rhythmic bladder contraction-inhibiting time. As the solvent for administration of the test compounds, 5% xylitol was used for test Compounds 1, 2, 3, 4, 5, 11, 12, 21, 23 and 24; physiological saline was used for test Compounds 6, 9, 10, 13, 14, 15, 16, 17, 18, 19, 20 and 22; 10% ethanol solution was used for test Compound 7; and 10% DMSO solution was used for test Compound 8. The solvents 5% xylitol solution, 10% ethanol solution and 10% DMSO solution, each of which is said to somewhat influence on the rhythmic bladder contraction-inhibiting time, were also tested at the dose of 1 ml/kg. The results are shown in Table 9. Any of the tested compounds exhibited rhythmic bladder contraction-inhibiting action when compared with the corresponding solvent used.

**Table 9**

| Test Compound | Dose | n | Mean Rhythmic Bladder Contraction-inhibiting Time (min) |
|---|---|---|---|
| 5% xylitol | 1 ml/kg | 4 | 1.3 |
| Compound 1 | 30 mg/kg | 4 | 6.2 |
| Compound 2 | 10 mg/kg | 4 | 4.5 |
| Compound 3 | 20 mg/kg | 4 | 5.0 |
| Compound 4 | 10 mg/kg | 4 | 13.9 |
| Compound 5 | 30 mg/kg | 4 | 5.7 |
| Compound 11 | 10 mg/kg | 4 | 12.4 |
| Compound 12 | 0.3 mg/kg | 5 | 14.6 |
| Compound 21 | 10 mg/kg | 4 | 10.0 |
| Compound 23 | 10 mg/kg | 4 | 9.2 |
| Compound 24 | 10 mg/kg | 4 | 2.3 |
| | | | |

| Saline | | | |
|---|---|---|---|
| Compound 6 | 3 mg/kg | 6 | 24.1 |
| Compound 9 | 1 mg/kg | 6 | 7.3 |
| Compound 10 | 10 mg/kg | 4 | 7.4 |
| Compound 13 | 1 mg/kg | 4 | 6.5 |
| Compound 14 | 3 mg/kg | 4 | 12.3 |
| Compound 15 | 1 mg/kg | 6 | 10.7 |
| Compound 16 | 0.3 mg/kg | 4 | 15.0 |
| Compound 17 | 1 mg/kg | 5 | 7.4 |
| Compound 18 | 1 mg/kg | 4 | 6.6 |
| Compound 19 | 1 mg/kg | 4 | 7.7 |
| Compound 20 | 1 mg/kg | 4 | 11.1 |
| Compound 22 | 10 mg/kg | 3 | 1.4 |
| | | | |
| 10% Ethanol | 1 ml/kg | 5 | 1.5 |
| Compound 7 | 5 mg/kg | 5 | 17.1 |
| | | | |
| 10% DMSO | 1 ml/kg | 4 | 3.5 |
| Compound 8 | 10 mg/kg | 4 | 19.5 |

## Claims

1. A therapeutic or prophylatic agent for frequent urination or urinary incontinence, comprising as an effective ingredient a quinolinomorphinan derivative of the Formula (I): 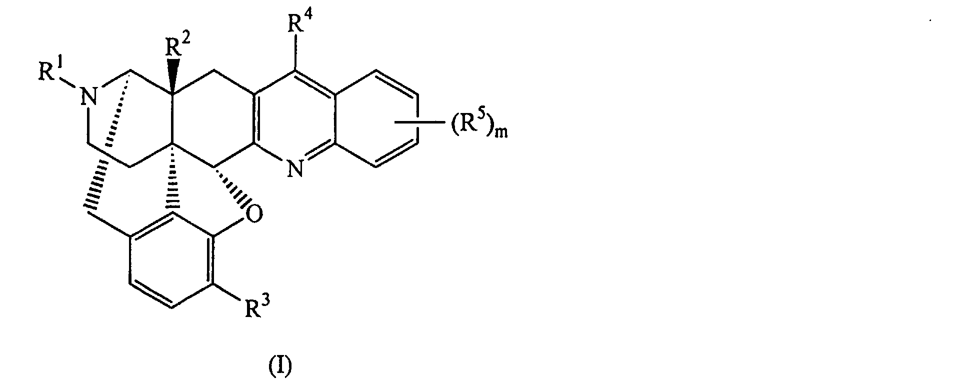 (wherein R¹ is hydrogen, C₁-C₅ alkyl, C₄-C₇ cycloalkylalkyl, C₅-C₇ cycloalkenylalkyl, C₆-C₁₂ aryl, C₇-C₁₃ aralkyl, C₂-C₇ alkenyl, C₁-C₅ alkanoyl, furan-2-ylalkyl (wherein the number of carbon atoms in the alkyl moiety is 1 to 5), or thiophene-2-ylalkyl (wherein the number of carbon atoms in the alkyl moiety is 1 to 5);
R² and R³ independently are hydrogen, hydroxy, C₁-C₅ alkoxy, C₁-C₅ alkanoyloxy, C₇-C₁₃ aralkyloxy or C₇-C₁₃ arylcarbonyloxy;
m is an integer of 0 to 4;
R⁵(s) is(are) substituent(s) on the benzene ring and independently represent(s) R¹⁸, or two R⁵s on adjacent carbon atoms cooperatively represent a fused ring structure A (with the proviso that the remaining 0 to 2 R⁵s independently represent R¹⁸ or a pair of R⁵s represent another fused ring structure A),
wherein said ring structure A is benzo, indeno, naphtho or pyrido, or C₅-C₇ cycloalkeno, each of which is substituted with 0 to 4 R⁹s, or non-substituted dioxoleno;
R⁹ and R¹⁸ represent (1) independently fluoro, chloro, bromo, iodo, nitro, hydroxy, C₁-C₅ alkyl, C₁-C₅ alkoxy, isothiocyanato, trifluoromethyl, trifluoromethoxy, cyano, phenyl, C₁-C₃ hydroxyalkyl, SR⁶, SOR⁶, SO₂R⁶, (CH₂)ₖCO₂R⁷, SO₂NR⁷R⁸, CONR⁷R⁸, (CH₂)ₖNR⁷R⁸, or (CH₂)ₖN(R⁷)COR⁸ (wherein k is an integer of 0 to 5, R⁶ is C₁-C₅ alkyl, R⁷ and R⁸ independently are hydrogen, C₁-C₅ alkyl or C₄-C₆ cycloalkylalkyl), and/or (2) R⁹ and R¹⁸ on adjacent carbon atoms via the fused ring moiety cooperatively form R⁹-R¹⁸ which represents a bridging structure selected from the group consisting of ethano, propano and *o*-benzeno;
R⁴ is hydrogen, C₁-C₅ alkyl, C₁-C₅ hydroxyalkyl, C₆-C₁₂ aryl (which may be substituted with one or more substituents R¹⁷), NR¹⁰R¹¹, OR¹², COOR¹³ or CONR¹⁴R¹⁵, or R⁴ and R⁵ which substitutes on the peri-position cooperatively form R⁴-R⁵ that represent a bridging structure selected from the group consisting of N(R¹⁶)CO, N(R¹⁶)C(=NH), N(R¹⁶)CH₂, *o*-benzeno, ethano, propano and butano; R¹⁷ is fluoro, chloro, bromo, iodo, nitro, amino, hydroxy, C₁-C₅ alkyl, C₁-C₅ alkoxy, C₁-C₅ alkanoyloxy, trifluoromethyl, trifluoromethoxy or cyano; R¹⁰, R¹¹, R¹² and R¹⁶ independently are hydrogen, C₁-C₅ alkyl, C₄-C₇ cycloalkylalkyl, C₇-C₁₃ aralkyl or C₁-C₅ alkanoyl, and R¹³, R¹⁴ and R¹⁵ independently are hydrogen, C₁-C₅ alkyl, C₆-C₁₂ aryl or C₇-C₁₃ aralkyl)
or a pharmaceutically acceptable acid addition salt thereof.

2. A therapeutic or prophylatic agent for frequent urination or urinary incontinence according to claim 1, wherein in Formula (I), R⁴ is hydrogen, C₁-C₅ alkyl or NR¹⁰R¹¹, or R⁴ and R⁵ which substitutes on the peri-position cooperatively form R⁴-R⁵ that represent a bridging structure N(R¹⁶)CO (wherein R¹⁰, R¹¹ and R¹⁶ represent the same meanings as in claim 1).

3. A therapeutic or prophylatic agent for frequent urination or urinary incontinence according to claim 2, wherein in Formula (I), R¹ is C₁-C₅ alkyl, C₄-C₇ cycloalkylalkyl, C₇-C₁₃ aralkyl, furan-2-ylalkyl (wherein the number of carbon atoms in the alkyl moiety is 1 to 5), or thiophene-2-ylalkyl (wherein the number of carbon atoms in the alkyl moiety is 1 to 5).
